# EUROPEAN PATENT SPECIFICATION

(11) **EP 1 945 233 B1**
(45) Date of publication and mention of the grant of the patent: **29.03.2017**
(21) Application number: 06836421.5
(22) Date of filing: 19.10.2006
(51) Int. Cl.: A61K 33/42, A61K 31/19, A61K 45/06, A61P 19/08, A61L 24/02, A61L 27/12, A61L 27/46, A61L 27/50, A61L 27/58

(54) **DUAL-PHASE CEMENT PRECURSOR SYSTEMS FOR BONE REPAIR**
DOPPELPHASEN-ZEMENT-VORLÄUFERSYSTEME ZUR KNOCHENREPARATUR
SYSTEMES PRECURSEURS DE CIMENT A DEUX PHASES POUR REPARER DES OS

(30) Priority: 21.10.2005 US 728888 P; 18.10.2006 US 550586
(43) Date of publication of application: 23.07.2008
(73) Proprietor: ADA FOUNDATION, Chicago, IL 60611 (US)
(72) Inventor: CHOW, Laurence, C., Gaithersburg, MD 20899 (US); TAKAGI, Shozo, Gaithersburg, MD 20899 (US)
(74) Representative: de Bresser, Sara Jean
(86) International application number: PCT/US2006/041034
(87) International publication number: WO 2007/047921

(56) References cited:
- EP-A1- 0 639 366
- US-A- 5 900 254
- US-B1- 6 652 875
- US-B2- 6 521 246

## Description

### CROSS REFERENCE TO RELATED APPLICATION

This application claims priority to prior U.S. provisional application Serial No. 60/728,888, filed October 21, 2005 and to prior U.S. patent application no. 11/550,586, filed October 18, 2006. Additionally, this application contains subject matter that in some embodiments is related to some of the subject matter described in U.S. provisional application Serial No. 60/728,838, filed October 21, 2005, and entitled "Biocompatible Endodontic Materials," and in U.S. patent application Serial No. 11/550543, entitled "Dental and Endodontic Filling Materials and Methods," filed on October 18, 2006.

### STATEMENT OF FEDERALLY SPONSORED RESEARCH

The invention was made in the course of research supported at least in part by Grant DE11789 from the National Institute of Dental and Craniofacial Research and carried out at the National Institute of Standards and Technology. The U.S. government may have certain rights to the invention.

### TECHNICAL FIELD

Generally, the invention is in the field of cements that are useful in connection with bone repair procedures. The invention provides dual-phase cement precursor systems in which the cement precursors take the form of first and second precursor phases. The phases initially are separate, but a cement suitable for bone repair procedures may be formed upon blending of the first and second phases.

### BACKGROUND OF THE INVENTION

Several types of self-hardening calcium phosphate compositions have been studied (Brown and Chow, A New Calcium Phosphate Water Setting Cement, pp. 352-379 in Brown, Cements Research Progress, American Ceramic Society, OH, 1986; Ginebra et al., Setting Reaction and Hardening of an Apatitic Calcium Phosphate Cement, J. Dent. Res. 76:905-912, 1997; Constantz et al., Histological, Chemical, and Crystallographic Analysis of Four Calcium Phosphate Cements in Different Rabbit Osseous Sites, J Biomed Mater. Res. [Appl. Biomater.] 43:451-461, 1998; Miyamoto et al., Histological and Compositional Evaluations of Three Types of Calcium Phosphate Cements When Implanted in Subcutaneous Tissue Immediately After Mixing, J. Biomed. Mater. Res. [Appl. Biomater.] 48:36-42, 1999*;* Lee et al., Alpha-BSM(R): A Biomimetic Bone Substitute and Drug Delivery Vehicle, Clin. Orthop Rel. Res. 367:396-405, 1999. Because of its chemical and crystallographic similarity to the carbonated apatitic calcium phosphate mineral found in human bones and teeth, hydroxyapatite has been one of the most often used restorative materials for the repair of human hard tissues

One of the calcium phosphate compositions, developed by Brown and Chow in 1986 and named calcium phosphate cement, or CPC, self-hardens to form hydroxyapatite as the primary product. The term "self-harden" refers to the paste being able to harden by itself. For example, the CPC paste can be placed into a bone cavity, and can self-harden after contact with an aqueous medium. CPC typically may be composed of particles of tetracalcium phosphate (TTCP: Ca₄(PO₄)₂O) and dicalcium phosphate anhydrous (DCPA: CaHPO₄) that react in an aqueous environment to form solid hydroxyapatite, Ishikawa et al., Reaction of Calcium Phosphate Cements with Different Amounts of Tetracalcium Phosphate and Dicalcium Phosphate Anhydrous, J. Biomed. Mater: Res. 46:504-510, 1999*;* Matsuya et al., Effects of Mixing Ratio and Ph on The Reaction Between Ca4[PO4]2O and CaHPO4, J. Mater. Sci.: Mater. in Med. 11:305-311, 2000*;* Takagi et al., Morphological and Phase Characterizations of Retrieved Calcium Phosphate Cement Implants, J. Biomed. Mater. Res. [Appl. Biomater.] 58:36-41, 2001*.* Calcium phosphate compositions (such as CPC) are highly promising for a wide range of clinical uses due to their excellent biocompatibility, osteoconductivity and bone replacement capability. For example, CPC has been studied for use in the reconstruction of frontal sinus and augmentation of craniofacial skeletal defects (Shindo et al., Facial Skeletal Augmentation Using Hydroxyapatite Cement, Arch. Otolaryngol. Head Neck. Surg., 119:185-190, 1993), endodontics (Sugawara et al., In vitro Evaluation of the Sealing Ability of a Calcium Phosphate Cement When Used as a Root Canal Sealer-Filler, J. Endodont. 16:162-165, 1990*),* and root canal applications (Chohayeb et al., Evaluation of Calcium Phosphate as a Root Canal Sealer-Filler Material, J. Endodont. 13:384-387,1987*).*

Most of the presently available calcium phosphate cements are mixed with an aqueous solution prior to use. EP 0639366, for example, discloses a process for producing hydroxyapatite cement by reacting a dry powder comprising a calcium salt and calcium phosphate with an aqueous solution prior to use. Accordingly, the ability of the surgeon to properly mix the cement and then place the cement paste into a bone defect within the prescribed time prior to cement hardening is a crucial factor in achieving optimum results. The art thus has recognized the desirability of providing pre-mixed cement pastes that are stable as provided but that harden only after being introduced to the bone defect and positioned appropriately. Pre-mixed self- hardening cements may be formulated by combining glycerol, sodium phosphate, hydroxypropyl methyl cellulose and calcium phosphate cement powders, as described in Takagi et al., "Properties of premixed calcium phosphate cement pastes," J. Biomed Mater Res. [Applied Biomater] 678:689 - 696 (2003). The hydroxypropyl methyl cellulose and sodium phosphate used in such pastes are believed to improve paste cohesiveness and accelerate cement hardening, respectively. The hardening times of the forgoing cements are about 60 minutes, which is much longer then 5- to 30-minute setting time desired in many cases.

Organic acids, such as glycolic, citric, tartaric, malonic, malic, maleic, and so forth, may be used as setting accelerators instead of sodium phosphate. In such cases, the pre-mixed cements can harden in significantly shorter times (10 minutes to 35 minutes) (Chow et al., "Rapid-Hardening, Pre-mixed Calcium phosphate cement pastes," Abs. No. 844, J. Dent. Res., Spec. Iss. A82 (2003)). The rapid hardening of these pre-mixed pastes is due to formation of carboxyl/calcium complexes, rather than the formulation of hydroxyapatite, which is the mechanism responsible for cement hardening in most conventional calcium phosphate cements. Despite the lack of hydroxyapatite formulation, several carboxylic acid/calcium phosphate cements had been reported to produce excellent bone defect repair results *in vivo.*

A third type of pre-mixed calcium phosphate cement has been reported (Carey et al., "Premixed Rapid-Setting Calcium Phosphate Composites for Bone Repair," Biomaterials 24:5002-14 (2005)). The cement hardening in these pre-mixed cements results from formulation of a hard hydrogel produced by a reaction between chitosan, a water soluble polymer, and an alkaline compound such as tetracalcium phosphate or calcium hydroxide. After initial hardening, further reactions between calcium phosphate salts form hydroxyapatite as a major end product in the cement.

To provide stability, the heretofore described cements are formulated as non-aqueous pre-mixed materials. Cement hardening does not begin until these precursors are placed into a bone defect, whereupon water from surrounding tissues enters into the cement. These cements, while often possessing excellent physical properties, sometimes can be limited in utility. Cement hardening in the interior of the cement mass may be slow under some clinical bone grafting conditions, for instance, wherein the amount of water available from the tissues is limited, or wherein the interior of the cement is more than several millimeters away from the nearest graft-tissue interface. Additionally, such cements typically are required to be formulated to be able to react extremely rapidly when exposed to moisture. Such formulations typically do not have a long shelf life, in light of the difficulties inherent in excluding moisture during manufacture and storage.

### SUMMARY OF THE INVENTION

Generally, the invention provides a dual-phase cement precursor system comprising first and second discrete pastes, said first paste comprising a first calcium phosphate compound and said second paste comprising a second calcium phosphate compound, said first and second pastes being separate, said first and second pastes forming a biocompatible cement upon mixing, characterised in that one of said first and second pastes being aqueous and the other of said first and second pastes being at least substantially non-aqueous, whereby trace amounts of moisture may be present in said substantially non-aqueous paste. The pastes themselves are not cements, but they may be combined to form a biocompatible cement that is useful in connection with bone repair procedures. In preferred embodiments, the cements thus formed are highly biocompatible, osteoconductive, and bioresorbable. A number of different but related and non-mutually exclusive cement chemistries may be employed in connection with the invention. For instance, in preferred embodiments, the cement may be a carboxyl/calcium cement, a hydrogel-forming polymer cement, or a calcium phosphate cement. One of the two pastes is aqueous, to allow the cement to set without the need to uptake water from surrounding tissue, and the other is at least substantially non-aqueous, whereby trace amounts of moisture may be present.

Also encompassed by the invention are various kits and methods. A kit comprises the heretofore described cement precursor system, in one of its various embodiments, and a dispensing device. The invention also encompasses the systems described herein for use in a method for bone repair, the method comprising providing a cement precursor system and applying a blend of the first and second precursor pastes to an area where bone repair is desired.

Further attributes of the preferred embodiments of the invention are described hereinbelow.

### DETAILED DESCRIPTION OF THE PREFERRED EMBODIMENTS

The cements that may be prepared from the cement precursors in accordance with the present invention are useful in connection with bone repair, by which is contemplated any bone grafting or other procedure used to correct defective or incorrectly formed bones, damages bones, and the like. The bone repair procedure may be performed in any animal, such as a human.

It is contemplated that a cement is a material that will set up, or harden, over a period of ninety minutes or less, when the cement is used at room temperature (20 - 25° C). Cement precursors are materials that in ordinary use themselves are not cements, but that may be blended with another precursor to form a cement. The cement precursors are provided in the form of a dual-phase cement precursor system, by which is contemplated a system that includes at least two (but optionally additional) precursor phases. Each of the phases comprises a compound or composition, and each phase in the dual-phase system is different from at least one other phase in the system. The phases themselves are not cements, in that the phases themselves do not set to form a hard material in ordinary use. Rather, when the phases are combined, a cement is formed thereby.

The system generally includes the two phases, which may be provided together in a container that is equipped to keep the phases separate until use. Any suitable container may be used in conjunction with the invention, and thus, for instance, the container may be any appropriate box, or bag, or package. In some cases, the container may be an appropriately configured syringe. The container may contain separate vials for the precursor phases, or separate compartments for the phases.

Preferably, the system is provided in the form of a kit, the kit including the dual phase cement precursor system and an appropriate mixing device. The mixing device may be conventional, or may otherwise be a device suitable for use in conjunction with the cement precursor systems taught. The prior art has provided a microdispenser with a static mixing tip, the mixing tip comprising an auger-like structure that allows the two phases to be blended rapidly and subsequently to be applied to the desired area. An example of such a device is the Dual-barrel 9 mL Micro Dispensing System by Tah Industries, Robinsville, N.J. The invention contemplates the use of this device, or an analogous device that is specifically designed for medical usage. In some embodiments, the microdispensers may include a region that serves as the container for the phases, by providing separate holding chambers for the first and second phases.

Each phase preferably is sufficiently stable to permit transport and reasonable storage prior to use. Stability may be measured by any technique or using any criteria deemed appropriate. In accordance with one such technique, a sample of the material or materials constituting the phase is heated to a temperature of 50° C, and held at this temperature for seven days.The material then is used in the formation of a cement, and the setting time of the cement is evaluated as compared with the original setting time of a similar cement made without thermal treatment of either of the phases. If the setting time of the cement made with the thermally treated phase is approximately equal to the setting time of the similar cement, the phase may be deemed suitably stable for use in conjunction with the present invention. The invention is not limited to cement precursor systems that meet this criterion; rather, the foregoing is provided to illustrate one of but many possible methods for evaluating stability.

The pastes themselves may be in any suitable form, so long one is aqueous. The water present in the aqueous paste generally should be present as liquid water, although it is contemplated in some embodiments that water may be present solely in the form of hydrates of solid materials. The other paste is a non-aqueous or substantially non-aqueous solid paste, or may be a liquid non-aqueous or substantially non-aqueous paste. By "substantially non-aqueous is contemplated that essentially no hydrated or liquid water is present in the paste. It is contemplated that in a substantially non-aqueous paste there will be trace amounts of moisture present, such as moisture that is unavoidably present notwithstanding reasonably prudent steps to exclude such moisture. In some embodiments, a liquid non-aqueous paste is provided. The liquid can be any suitable room temperature liquid, examples of which include glycerin, glycerol, ethanol, propanol, certain polyethylene glycols, and propylene glycol. Glycerin is deemed particularly preferred, in light of its biocompatibility and complete miscibility with water.

In forming a cement, the phases may be blended in any suitable amounts. When each phase is a liquid, preferably the phases are formulated such that the volumetric ratio of mixing ranges from 0.1- 10, more preferably, 0.25-4.

The cement precursors may be any material suitable for use in forming a biocompatible cement, the term "biocompatible" (when used in conjunction with a cement) contemplating a cement that is not rejected by soft tissue or hard tissue when used *in vivo* in the intended application. Numerous cement chemistries may be used in conjunction with the convention. In a first preferred embodiment, a calcium phosphate cement is employed. Calcium phosphate cements are deemed to be those cements in which at least two dissimilar calcium phosphate materials are present respectively in the two precursor phases, and in which, upon blending, a complex or mixture or third calcium phosphate material (hydroxyapatite, for instance) is formed. In such cements, some of the third material may be present in one or both of the precursor phases. In a second preferred embodiment, a carboxyl/calcium cement is employed. In such cements, one of the phases includes a carboxylic acid or carboxylate, and the other phase includes a calcium compound, and i n which, upon blending of the phases, a carboxyl/calcium complex is formed. Additional materials may be present; for instance, the first phase may include a calcium compound in addition to the carboxylic acid or carboxylate, so long as the phase is stable and does not form a hardened cement in ordinary use prior to blending with the second phase. For instance, the first phase may include a calcium phosphate compound that is sufficiently acidic that it does not dissociate and thus does not react with the carboxylic acid or carboxylate, even in the presence of any water present in this phase. In a third preferred embodiment, a hydrogel cement is employed. A hydrogel cement is deemed to be a cement in which a hydrogel-forming polymer and a hydrogel former are present in at least one of the precursor phases. In many (but not all) embodiments, the hydrogel-forming polymer is present in one of the phases, and the hydrogel former is present in the other phase. Upon blending of the phases, a hardened hydrogel is formed. These cement chemistries are not mutually exclusive, and it is possible that two or more mechanisms of cement formation may be present in a given cement precursor system. Likewise, other cement chemistries may be employed instead of or in addition to the foregoing.

Where a carboxyl/calcium cement is employed, one of the phases contains a carboxylic acid or the salt of a carboxylic acid. Any suitable biocompatible carboxylic acid or salt may be used in conjunction with the invention, and accordingly the term "carboxyl" is deemed to include both the acid and the salt forms of organic acids. In preferred embodiments, the carboxylic acid or salt is at least one acid that is selected from among glycolic, citric, tartaric, glycerophosphoric, malonic, malic, and maleic acids. It is not necessary that the pH of the phase that includes the carboxylic acid or salt be in the acidic range. More generally, any other suitable acid may be used in conjunction with the invention.

In such systems, the other phase contains a calcium compound. Any suitable calcium compound may be used in conjunction with this embodiment of the invention. In preferred embodiments, the calcium compound is a calcium phosphate having a Ca/P ratio ranging from 0.5 - 2.0. Alternatively, or in addition thereto, the calcium compound may be a suitable calcium salt, or any suitable calcium compound that is sparing soluble in acid. Exemplary calcium compounds suitable for use in conjunction with the invention include tetracalcium phosphate (TTCP), dicalcium phosphate anhydrous (DCPA), dicalcium phosphate dihydrate (DCPD), monocalcium phosphate anhydrous (MCPA), monocalcium phosphate monohydrate (MCPM), alpha-tricalcium phosphate (alpha-TCP), beta tricalcium phosphate (beta-TCP), hydroxyapatite (HA), amorphous calcium phosphate (ACP), octacalcium phosphate (OCP), calcium deficient hydroxyapatite, carbonate-containing hydroxyapatite (CHA), fluoride-containing hydroxyapatite (FHA), calcium lactate, calcium sulfate, calcium gluconate, calcium lactate gluconate, calcium glycerophosphate, calcium silicate, calcium hydroxide, and other biocompatible calcium compounds with a solubility of at least about 2 wt. % in the acid environment. Generally, calcium compounds that are biocompatible and that form a suitable cement may be used. The selection of a particular calcium compound may be based on numerous factors, including for instance the reactivity of the compound with the selected acid, and also the overall acid and base contents of the cement, and the desired end cement products.

Generally, in each phase, the acid and calcium compound respectively may be present in any suitable amounts. The acid is preferably present in an aqueous phase in an amount ranging from about or exactly 1 to about or exactly 75 percent by weight of the total phase, more preferably, about or exactly 5 to about or exactly 35 percent. The calcium compound may or may not be present in an aqueous phase. Further details concerning the preferred ranges of calcium may be found in the examples set forth herein below.

When the calcium and carboxyl phases are combined, a cement is formed. The cement is formed by calcium in combination with a dissociated carboxylic acid residue (RCOO} In some embodiments, calcium may be present in the carboxyl phase of the cement, so long as the cement is stable (for instance, at sufficiently low pH). In such embodiments, some of the calcium from the carboxyl phase may contribute to the formation of the carboxyl/calcium setting cement. In such embodiments, the calcium-containing phase may contribute to cement formation in part by causing a rise in pH. Generally, in these embodiments, the pH of the cement should range from about 2-9 prior to setting.

In the case of hydrogel cements, the phases collectively include a hydrogel-forming polymer and the hydrogel former. In some embodiments, one of the phases includes a hydrogel-forming polymer and the other phase includes the hydrogel former. In other embodiments, the polymer and former initially are present together in one phase, that phase being at least substantially non-aqueous. In these embodiments, the other phase is an aqueous phase. The hydrogel- forming polymer may be any suitable polymer that is biocompatible and that will form a hardened hydrogel upon contact with the hydrogel former. Particularly preferred hydrogel-forming polymers include chitosan and biocompatible chitosan derivatives, alginic acid and alginic acid derivatives, in particular alginates, and pectinic acid and pectinic acid derivatives, in particular pectinates. Suitable alginic acid derivatives include sodium alginate, propylene glycol alginate, and other soluble alginate salts. Suitable pectinic acid derivatives include sodium pectinate and other suitable pectinate salts.

When the polymer is chitosan, the polymer will form a hydrogel upon exposure to alkalinity. Accordingly, any suitable alkaline agent, such as sodium hydroxide, potassium hydroxide, or calcium hydroxide, and calcium, sodium, or potassium salts of phosphoric acid or silicic acid, may be used as the hydrogel former. However, calcium-containing materials, in particular, calcium hydroxide, calcium phosphate, and calcium silicate, are preferred in bone cement applications. When the hydrogel-forming polymer includes alginic or pectinic acid or a derivative thereof, hydrogel formation occurs via formation of complexes with calcium, and thus the hydrogel former should be a material that includes calcium. Particularly preferred materials include the calcium compounds referenced hereinabove, such as TTCP, DCPA, and the like. Other suitable calcium compounds include calcium oxide, calcium chloride, calcium lactate, calcium glutonate, calcium silicate, and calcium carbonate. Again, as described hereinabove, the polymer and hydrogel former may be present in the precursor phases in any suitable amounts. Generally, each material may be present in amounts of from about or exactly 1 to about or exactly 75 percent by weight of the total phase, more preferably, about or exactly 5 to about or exactly 35 percent by weight of the total phase.

The cement comprises a calcium phosphate cement. In such cements, a calcium phosphate precursor is present in one of the phases, and a dissimilar calcium phosphate precursor is present in the other one of the phases. When blended, a calcium phosphate complex, or third calcium phosphate, solidifies to form the cement. In many cases, hydroxyapatite or DCPD is formed. The phases need not include only a single calcium phosphate material, and thus, for instance, the phases each may include multiple calcium phosphate materials, and some of the third calcium phosphate material may be present initially in either or both of the phases.

Any suitable calcium phosphate compounds may be used in the respective first and second phases, and many of the heretofore discussed calcium compounds are deemed particularly suitable. Generally, it is preferred that the Ca/P ratio ranges from 0.5 to 2.0 in each phase. In some embodiments, particularly when it is desired to form hydroxyapatite, one of the phases includes a calcium phosphate in which the Ca/P ratio is less than 5/3, and the other includes a calcium phosphate compound in which the Ca/P ratio is greater than 5/3. The Ca/P ratio in hydroxyapatite is 5/3, and it is believed that providing calcium and phosphate in both greater and lesser amounts will drive formation of hydroxyapatite. It is not necessary to employ two such phases, especially if a setting accelerator is used (as described hereinbelow). In some embodiments, the Ca/P ratio in one of the pastes is equal to 5/3. In the formation of hydroxyapatite with the heretofore described calcium phosphate cements, the formation of hydroxyapatite can proceed slowly if the cement is initially formed at a pH above about 8, and if the selection of precursors for such a cement would provide a pH of 8 or above, use of a setting accelerator is preferred. In some embodiments, one may choose the overall Ca/P in order to cause formation of a different calcium phosphate in the resulting cement, such as DCPA (as set forth in Example 42 below) or DCPD.

These cement chemistries are not mutually exclusive, and it is thus contemplated that a cement precursor system may include materials that, when blended, form a cement that has attributes of two or more of the heretofore described cements.

The nature of the compounds and functional materials present in the cements is not limited to the heretofore described ingredients, but to the contrary any other suitable osteoconductive, bioactive, bioinert, or other functional materials may be used in conjunction with the invention. When used, these optional ingredients, may be present in any amounts suitable for their intended purposes. For instance, particularly in the case of the calcium phosphate cements, one or both cement precursor phases may include a setting accelerator, such as phosphoric acid, hydrochloric acid, sulfuric acid, oxalic acid, and salts thereof, and sodium phosphate, potassium phosphate, and sodium fluoride. In some embodiments, some of the calcium phosphate materials themselves may promote setting; for instance, MCPM and certain nano-sized calcium phosphate materials may promote setting of the cement. Any other suitable setting accelerator may be used in conjunction with the present invention. Setting accelerators are described in more detail in Chow et al., U.S. Patent Application Publication No. 20050074415, published April 7, 2005.

In some embodiments, one of the cement precursors includes an osteoinductive protein, by which is contemplated any protein that is useful in assisting in or inducing bone formation. Osteoinductive proteins are deemed particularly suitable for use in conjunction with the carboxyl/calcium cement systems because, at least for many known osteoinductive proteins, such proteins may denature at an alkaline pH.

Another optional ingredient is a filler, such as a radio opaque filler. The radio opaque filler may, for instance, be a suitable bismuth, barium, or iodide compound, such as barium sulfate or bismuth hydroxide. Other suitable fillers include bioglass, silicas, alumina, biphasic calcium phosphate, calcium silicate, calcium sulfate, granular calcium phosphate ceramics, Portland cement, and the like.

A medicament, such as zinc, magnesium, or any other suitable medicament may be included in one or both of the phases of the cement precursors.

Either or both of the phases may include a material that is intended to affect the viscosity, cohesiveness, or injectability of the phases. Any suitable biocompatible ingredient, such as hydroxypropyl methyl cellulose (HPMC) or the like may be employed in conjunction therewith.

In some embodiments, a macropore forming material may be used. As disclosed, for instance, in prior U.S. patent nos. 7,018,460 and 6,955,716, a macropore forming material, such as mannitol, is useful in forming a macropores, or pores having a size greater than 150 microns. Such pores are sometimes deemed desirable and that they create a structure that may be useful in promoting growth of soft tissue in or near the region of these cements.

Also as described in prior U.S. patent nos. 7,018,460 and 6,955,716, in some embodiments, one or more strength-enhancing components, such as fibers, meshes, or the like, may be used. Such components may be resorbable or non-resorbable.

The following Examples are provided to illustrate the invention, but should not be construed as limiting the invention in scope. All of these Examples describe dual-phase cement precursor systems in which two liquid pastes were prepared, the pasted being denoted as Paste 1 and Paste 2. An asterisk symbol ("*") denotes that both pastes are aqueous, and the absence of an asterisk symbol denotes that one paste is aqueous and the other paste is non-aqueous.

### EXAMPLES

Examples 1-25 describe carboxyl/calcium cement systems. Examples 1a, 1b, 7, 8, 20, 26, 29, 33, 34, 38-42, and 43-45 describe formulations which are encompassed by the present invention.

### EXAMPLE 1

Paste 1 was prepared by blending 3.0 g of barium sulfate (BaSO₄) and 0.12g hydroxypropyl methyl cellulose (HPMC) into 1.5 mL of an aqueous solution (8.5 M) of glycolic acid. Paste 2 was composed of 3g of a calcium phosphate cement (CPC) mixture (containing 73 % tetracalcium phosphate (TTCP) and 27 % dicalcium phosphate anhydrous (DCPA)) and 1.0 g of glycerin. Approximately equal volumes of the two pastes were dispensed and homogeneously mixed using a dual-barrel micro dispenser (1:1 volume ratio) equipped with a static mixer in the delivery tip. The combined pastes hardened in less than 5 minutes at room temperature (21° C).

Both pastes were aged for ten days at 50°C. No measurable changes in paste consistency or setting reaction was observed. Diametral tensile strength (DTS) was evaluated under the methodology described in Lemaitre et al., "Setting, Hardening and Resorption of Calcium Phosphate Hydraulic Cements," Rev Stomatal. Chir. Maxiffofac. 1992;93:163-165. The DTS of 1-day set cement sample was 2.52 ± 0.37 MPa (n = 5). Without limiting the scope of the claims of the invention, in general a DTS in the range of 1.2 MPA to 3.5 MPa is deemed satisfactory, and accordingly this cement was deemed to be satisfactory.

### EXAMPLE 1a

Paste 1 was prepared by blending 2.5 g ofmonocalcium phosphate monohydrate (MCPM) into 1.0 ml of an aqueous solution (8.5 M) of glycolic acid. Paste 2 was composed of 2.5g of a calcium phosphate cement (CPC) mixture (containing 73 % tetracalcium phosphate (TTCP) and 27 dicalcium phosphate anhydrous (DCPA)) and 1.0 g of polyethylene glycol 400. Approximately equal volumes of the two pastes were dispensed and homogeneously mixed using a dual-barrel micro dispenser (1:1 volume ratio) equipped with a static mixer in the delivery tip. The combined pastes hardened about 5 minutes at room temperature (21° C). Diametral tensile strength (DTS) of 1-day set cement sample was 1.40 ± 0.02 MPa (n = 3).

### EXAMPLE 1b

Paste 1 was prepared by blending 2.54 g of monocalcium phosphate monohydrate (MCPM) into 1.0 ml of an aqueous solution (8.5 M) of glycolic acid. Paste 2 was composed of 2.5g of a tetracalcium phosphate (TTCP) and 1.0 g of polyethylene glycol 400. Approximately equal volumes of the two pastes were dispensed and homogeneously mixed using a dual-barrel micro dispenser (1:1 volume ratio) equipped with a static mixer in the delivery tip. The combined pastes hardened about 5 minutes at room temperature (21°C). Diametral tensile strength (DTS) of 1-day set cement sample was 1.84±0.08 MPa (n = 4).

### EXAMPLE2

Paste 1 was prepared by blending 3.0 g of barium sulfate into 1.5 mL of an aqueous solution (8.5 M) of tartaric acid. Paste 2 was composed of 3g of a calcium phosphate cement (CPC) mixture (containing 73 % tetracalcium phosphate (TTCP) and 27 % dicalcium phosphate anhydrous (DCPA)) and 1.2 g of glycerin. Approximately equal volumes of the two pastes were dispensed and homogeneously mixed using a dual-barrel micro dispenser (1:1 volume ratio) equipped with a static mixer in the delivery tip. The combined pastes hardened in less than 5 minutes at room temperature (21°C).

### EXAMPLE3

Paste 1 was prepared by blending 3.0 g of alumina (Al₂O₃) into 1.5 mL of an aqueous solution (8.5 M) of glycolic acid. Paste 2 was composed of 3g of a calcium phosphate cement (CPC) mixture (containing 73% tetracalcium phosphate (TTCP) and 27 % dicalcium phosphate anhydrous (DCPA)) and 1.1 g of glycerin. Approximately equal volumes of the two pastes were dispensed and homogeneously mixed using a dual-barrel micro dispenser (1:1 volume ratio) equipped with a static mixer in the delivery tip. The combined pastes hardened in approximately 5 minutes at room temperature (21°C).

### EXAMPLE4

Paste 1 was composed of 3.0 g of TTCP and 1.3 mL of water. Paste 2 was composed of 3g of glycolic acid granules and 1 g of glycerin. Approximately equal volumes of the two pastes were dispensed and homogeneously mixed using a dual-barrel micro dispenser (1:1 volume ratio) equipped with a static mixer in the delivery tip. The combined pastes hardened in 2 - 3 minutes at room temperature (21°C).

### EXAMPLE 5

Paste 1 was composed of 3.0 g of TTCP and 1.3 mL of water. Paste 2 was composed of 3g of maleic acid granules and 1 g of glycerin. Approximately equal volumes of the two pastes were dispensed and homogeneously mixed using a dual-barrel micro dispenser (1:1 volume ratio) equipped with a static mixer in the delivery tip. The combined pastes hardened almost instantly at room temperature (21°C).

### EXAMPLE6

Paste 1 was composed of 3.0 g of TTCP and 1.5 mL of water. Paste 2 was composed of 3g of citric acid granules and 1 g of glycerin. Approximately equal volumes of the two pastes were dispensed and homogeneously mixed using a dual-barrel micro dispenser (1:1 volume ratio) equipped with a static mixer in the delivery tip. The combined pastes hardened in approximately 30 minutes at room temperature (21°C).

### EXAMPLE 7

Paste 1 was composed of 4.0 g of monocalcium phosphate monohydrate (MCPM) and 1.5 mL of an aqueous solution (8.5 M) of glycolic acid. Paste 2 was composed of 1.5 g of calcium glycerophosphate, 1.5 g of calcium hydroxide, and 1 g of glycerin. Approximately equal volumes of the two pastes were dispensed and homogeneously mixed using a dual-barrel micro dispenser (1:1 volume ratio) equipped with a static mixer in the delivery tip. The combined pastes hardened instantly at room temperature (21°C).

### EXAMPLE 8

Paste 1 was composed of 3.0 g of TTCP and 1.5 mL of water. Paste 2 was composed of 2g of tartaric acid granules, 1g MCPM and 1.5 g of glycerin. Approximately equal volumes of the two pastes were dispensed and homogeneously mixed using a dual-barrel micro dispenser (1:1 volume ratio) equipped with a static mixer in the delivery tip. The combined pastes hardened almost immediately at room temperature (21°C).

### EXAMPLE 9*

Paste 1 was composed of 3.0 g ofbeta-tricalcium phosphate β-TCP) and 1.3 mL of water. Paste 2 was composed of 3g of Al₂O₃ and 1.5 mL of an aqueous solution (8.5 M) of glycolic acid. Approximately equal volumes of the two pastes were dispensed and homogeneously mixed using a dual-barrel micro dispenser (1:1 volume ratio) equipped with a static mixer in the delivery tip. The combined pastes hardened instantly at room temperature (21°C).

### EXAMPLE 10

Paste 1 was composed of 3g of Al₂O₃ and 1.5 mL of an aqueous solution (8.5 M) of glycolic acid. Paste 2 was composed of 3.0 g of alpha-tricalcium phosphate (α-TCP) and 1.4g polyethylene glycol 400. Approximately equal volumes of the two pastes were dispensed and homogeneously mixed using a dual-barrel micro dispenser (1:1 volume ratio) equipped with a static mixer in the delivery tip. The combined pastes hardened instantly at room temperature (21°C).

### EXAMPLE 11*

Paste 1 was composed of 3.0 g of commercially obtained hydroxyapatite (HA) (bio-Rad) and 2.5 mL of water. Paste 2 was composed of 3g of Al₂O₃ and 1.5 mL of an aqueous solution (8 M) of tartaric acid. Approximately equal volumes of the two pastes were dispensed and homogeneously mixed using a dual-barrel micro dispenser (1:1 volume ratio) equipped with a static mixer in the delivery tip. The combined pastes hardened in approximately 5 minutes at room temperature (21°C).

### EXAMPLE 12

Paste 1 was composed of 3g of BaSO₄ and 1.2 mL of an aqueous solution (8.5 M) of glycolic acid. Paste 2 was composed of 1.2 g of amorphous calcium phosphate (ACP) and 1.2 g glycerin. Approximately equal volumes of the two pastes were dispensed and homogeneously mixed using a dual-barrel micro dispenser (1:1 volume ratio) equipped with a static mixer in the delivery tip. The combined pastes hardened in 2 to 3 minutes at room temperature (21°C).

### EXAMPLE 13

Paste 1 was composed of 3g of BaSO₄ and 1.3 mL of an aqueous solution (8.5 M) of glycolic acid. Paste 2 was composed of 1.5 g of octacalcium phosphate (OCP) and 1.8 g glycerin. Approximately equal volumes of the two pastes were dispensed and homogeneously mixed using a dual-barrel micro dispenser (1:1 volume ratio) equipped with a static mixer in the delivery tip. The combined pastes hardened in less than 5 minutes at room temperature (21°C).

### EXAMPLE 14

Paste 1 was composed of 3g of BaSO₄ and 1.0 mL of an aqueous solution (14 M) of malonic acid. Paste 2 was composed of 2.9 g of a CPC mixture consisting of 3 moles of α-TCP and 1 mole of CaCO₃ (Ca/P molar ratio = 1.67) and 1.5 g glycerin. Approximately equal volumes of the two pastes were dispensed and homogeneously mixed using a dual-barrel micro dispenser (1:1 volume ratio) equipped with a static mixer in the delivery tip. The combined pastes hardened in approximately 50 minutes at room temperature (21°C).

### EXAMPLE 15

Paste 1 was composed of 3.1 g of BaSO₄ and 1.2 mL of an aqueous solution (8.5 M) of glycolic acid. Paste 2 was composed of 1.6 g of a CPC mixture consisting of 3 moles of dicalcium phosphate anhydrous (DCPA) and 2 moles of Ca(OH)₂ (Ca/P molar ratio = 1.67) and 1.4 g glycerin. Approximately equal volumes of the two pastes were dispensed and homogeneously mixed using a dual-barrel micro dispenser (1:1 volume ratio) equipped with a static mixer in the delivery tip. The combined pastes hardened in approximately 10 minutes at room temperature (21° C).

### EXAMPLE 16

Paste 1 was composed of 3 g of BaSO₄ and 1.2 mL of an aqueous solution (8.5 M) of glycolic acid. Paste 2 was composed of 1.6 g of a CPC mixture consisting of 3 moles of DCPA and 2 moles of Ca(OH)₂ (Ca/P molar ratio = 1.67) and 1.4 g glycerin. Approximately equal volumes of the two pastes were dispensed and homogeneously mixed using a dual-barrel micro dispenser (1:1 volume ratio) equipped with a static mixer in the delivery tip. The combined pastes hardened in approximately 10 minutes at room temperature (21°C).

### EXAMPLE 17*

Paste 1 was composed of 1.5 g of MCPM, 1.5 g of DCPD, 1.7 g of glycolic acid granules, and 3 mL of a solution saturated with respect to both MCPM and DCPD ([Ca] = 1.3 M, [P] = 4.4 M, pH= 1.9). Paste 2 was composed of 3g of a TTCP and 1.3 mL of water. Approximately equal volumes of the two pastes were dispensed and homogeneously mixed using a dual-barrel micro dispenser (1:1 volume ratio) equipped with a static mixer in the delivery tip. The combined pastes set instantly at room temperature (21°C).

### EXAMPLE 18*

Paste 1 was composed of 1.5 g of MCPM, 1.5 g of DCPD, 1.7 g of glycolic acid granules, and 3 mL of a solution saturated with respect to both MCPM and DCPD ([Ca] = 1.3 M, [P] = 4.4 M, pH= 1.9). Paste 2 was composed of 3g of a TTCP, 0.75 mL of water, and 0.75 g of glycerin. Approximately equal volumes of the two pastes were dispensed and homogeneously mixed using a dual-barrel micro dispenser (1:1 volume ratio) equipped with a static mixer in the delivery tip. The combined pastes set in less than 5 minutes at room temperature (21°C).

### EXAMPLE 19*

Paste 1 was composed of 3 g of DCPA and 1.4 mL of an aqueous solution (2 M) of citric acid. Paste 2 was composed of 3g of TTCP and 1.4 mL of water. Approximately equal volumes of the two pastes were dispensed and homogeneously mixed using a dual-barrel micro dispenser (1:1 volume ratio) equipped with a static mixer in the delivery tip. The combined pastes set almost immediately at room temperature (21°C). Diametral tensile strength of 1-day set cement sample was 4.18 ± 1.02 MPa (n = 5).

### EXAMPLE20

Paste 1 was composed of 3 g of DCPA and 1.4 mL of an aqueous solution (2 M) of citric acid. Paste 2 was composed of 3g of TTCP and 1.35 g of glycerin. Approximately equal volumes of the two pastes were dispensed and homogeneously mixed using a dual-barrel micro dispenser (1:1 volume ratio) equipped with a static mixer in the delivery tip. The combined pastes set in less than 10 minutes at room temperature (21°C). Diametral tensile strength of 1-day set cement sample was 3.86 ± 0.86 MPa (n = 5).

### EXAMPLE 21*

Paste 1 was composed of 3 g of DCPA and 1.4 mL of an aqueous solution (2 M) of citric acid. Paste 2 was composed of 3g of TTCP, 0.5 g of water and 1 g of glycerin. Approximately equal volumes of the two pastes were dispensed and homogeneously mixed using a dual-barrel micro dispenser (1:1 volume ratio) equipped with a static mixer in the delivery tip. The combined pastes set in less than 5 minutes at room temperature (21°C).

### EXAMPLE 22

Paste 1 was composed of 3 g of Ca(OH)₂ and 2 mL of water. Paste 2 was composed of 3 g of citric acid granules and 1 g of glycerin. Approximately equal volumes of the two pastes were dispensed and homogeneously mixed using a dual-barrel micro dispenser (1:1 volume ratio) equipped with a static mixer in the delivery tip. The combined pastes set in less than 5 minutes at room temperature (21°C).

### EXAMPLE 23*

Paste 1 was composed of 1.5 g of ground DCPA, 0.01 g of HPMC and 1.2 g of an aqueous solution (2M) of malic acid solution. Paste 2 was composed of 1.5 g of TTCP and 0.7 g of 1% HPMC aqueous solution. Approximately equal volumes of the two pastes were dispensed and homogeneously mixed using a dual-barrel micro dispenser (1:1 volume ratio) equipped with a static mixer in the delivery tip. The combined pastes set in less than 5 minutes at room temperature (21°C). Diametral tensile strength (DTS) of 1-day set cement sample was 3.48 ± 0.40 MPa (n = 5).

### EXAMPLE24

Paste 1 was composed of 2 g of MCPM, 1 g of an aqueous solution (8.5M) of glycolic acid solution and 0.1 g of glycerin. Paste 2 was composed of 3 g of Portland cement and 1.2 g of glycerin. Approximately equal volumes of the two pastes were dispensed and homogeneously mixed using a dual-barrel micro dispenser (1:1 volume ratio) equipped with a static mixer in the delivery tip. The combined pastes set in about 9 minutes at room temperature (21°C). Diametral tensile strength of 1-day set cement sample was 2.31 ± 0.65 MPa (n = 5).

### EXAMPLE25

Paste 1 was composed of 3 g of MCPM, 1.25 g of an aqueous solution (8.5M) of glycolic acid solution. Paste 2 was composed of 3 g of tricalcium silicate and 2 g of glycerin. Approximately equal volumes of the two pastes were dispensed and homogeneously mixed using a dual-barrel micro dispenser (1:1 volume ratio) equipped with a static mixer in the delivery tip. The combined pastes set in about 4 minutes at room temperature (21°C).

Examples 26-35 describe hydrogel cement systems

### EXAMPLE26

Paste 1 was prepared by blending 3.0 g of MCPM into 2.5 g of a chitosan lactate solution (15 % chitosan lactate+ 85 %water). Paste 2 was composed of 3g of a calcium phosphate cement (CPC) mixture (containing 73 % tetracalcium phosphate (TTCP) and 27 % dicalcium phosphate anhydrous (DCPA)) and 1.2 g of glycerin. Approximately equal volumes of the two pastes were dispensed and homogeneously mixed using a dual-barrel micro dispenser (1:1 volume ratio) equipped with a static mixer in the delivery tip. The combined pastes set in approximately 5 minutes at room temperature (21°C) forming a non-rigid hard mass. Diametral tensile strength of 1-day set cement sample was 2.15 ± 0.21 MPa (n = 5).

### EXAMPLE 27*

Paste 1 was prepared by blending 3.0 g of BaSO₄ into 1.5 g of a sodium alginate solution (20 % sodium alginate + 80 % water). Paste 2 was composed of 3g of a DCPA and 1.5 mL of a pH 2.1 solution saturated with respect to dicalcium phosphate dihydrate. Approximately equal volumes of the two pastes were dispensed and homogeneously mixed using a dual-barrel micro dispenser (1:1 volume ratio) equipped with a static mixer in the delivery tip. The combined pastes set in approximately 5 minutes at room temperature (21°C).

### EXAMPLE28

Paste 1 was composed of 2.5 g of MCPM, 1.8 g of a chitosan lactate solution (15 % chitosan lactate+ 85 %water), and 1 g of glycerin. Paste 2 was composed of 3g of calcium carbonate and 1 g of glycerin. Approximately equal volumes of the two pastes were dispensed and homogeneously mixed using a dual-barrel micro dispenser (1:1 volume ratio) equipped with a static mixer in the delivery tip. The combined pastes set in approximately 5 minutes at room temperature (21°C) forming a non-rigid hard mass.

### EXAMPLE29

Paste 1 was composed of 2.5 g of MCPM, 1.8 g of a chitosan lactate solution (15 % chitosan lactate+ 85 %water), and 1 g of glycerin. Paste 2 was composed of 3g of a calcium phosphate cement (CPC) mixture (containing 73 % tetracalcium phosphate (TTCP) and 27 % dicalcium phosphate anhydrous (DCPA)) and 1.5 g of glycerin. Approximately equal volumes of the two pastes were dispensed and homogeneously mixed using a dual-barrel micro dispenser (1:1 volume ratio) equipped with a static mixer in the delivery tip. The combined pastes set almost immediately at room temperature (21°C) forming a non-rigid hard mass.

### EXAMPLE30

Paste 1 was composed of 3 g of TTCP and 1.4 mL of water. Paste 2 was composed of 2 g of chitosan malate and 1 g of glycerin. Approximately equal volumes of the two pastes were dispensed and homogeneously mixed using a dual-barrel micro dispenser (1:1 volume ratio) equipped with a static mixer in the delivery tip. The combined pastes set almost instantly at room temperature (21°C) forming a non-rigid hard mass.

### EXAMPLE31

Paste 1 was composed of 3 g of CaCO₃ and 1.5 mL of water. Paste 2 was composed of 2 g of chitosan malate and 1 g of glycerin. Approximately equal volumes of the two pastes were dispensed and homogeneously mixed using a dual-barrel micro dispenser (1:1 volume ratio) equipped with a static mixer in the delivery tip. The combined pastes set in approximately 5 minutes at room temperature (21°C) forming a non-rigid hard mass.

### EXAMPLE32

Paste 1 was prepared by blending 3 g of MCPM into 3 g of a chitosan lactate solution (15 % chitosan lactate+ 85 %water). Paste 2 was composed of 3g of a Portland cement and 1.2 g of glycerin. Approximately equal volumes of the two pastes were dispensed and homogeneously mixed using a dual-barrel micro dispenser (1:1 volume ratio) equipped with a static mixer in the delivery tip. The combined pastes set in approximately 1 minutes at room temperature (21°C) forming a non-rigid hard mass. Diametral tensile strength (DTS) of 1-day set cement sample was 2.26 ± 0.75 MPa (n = 5).

### EXAMPLE33

Paste 1 was prepared by blending 1 g of DCPA and 1.2 g of barium sulfate into 1.1 g of a chitosan lactate solution (15 % chitosan lactate + 85 % water). Paste 2 was composed of 1.4 g of TTCP, 0.6 g of tricalcium silicate, 0.2 g of Na₂HPO₄, and 1.2 g of glycerin. Approximately equal volumes of the two pastes were dispensed and homogeneously mixed using a dual-barrel micro dispenser (1:1 volume ratio) equipped with a static mixer in the delivery tip. The combined pastes set in approximately 10 minutes at room temperature (21°C) forming a non-rigid hard mass. Diametral tensile strength of 1-day set cement sample was 2.06 ± 0.37 MPa (n = 5).

### EXAMPLE34

Paste 1 was prepared by blending 2 g of DCPA and 1 g of MCPM into 1.5 g of a chitosan lactate solution (5 % chitosan lactate + 95 % water). Paste 2 was composed of 3 g of TTCP and 1.7 g of glycerin. Approximately equal volumes of the two pastes were dispensed and homogeneously mixed using a dual-barrel micro dispenser (1:1 volume ratio) equipped with a static mixer in the delivery tip. The combined pastes set in approximately 20 minutes at room temperature (21°C) forming a non-rigid hard mass.

### EXAMPLE 35

Paste 1 was prepared by blending 3.0 g of TTCP into 1.5 g of a sodium alginate solution (20% sodium alginate+80% water), and 0.1 g of glycerin. Paste 2 was composed of 3g of a DCPA and 1.5 mL of a pH 2.1 solution saturated with respect to dicalcium phosphate dihydrate. Approximately equal volumes of the two pastes were dispensed and homogeneously mixed using a dual-barrel micro dispenser (1:1 volume ratio) equipped with a static mixer in the delivery tip. The combined pastes set in approximately 20 minutes at room temperature (21°C) forming a non-rigid hard mass.

Example 36-39 describe cement systems in which both carboxyl/calcium cements and hydrogel cements are employed.

### EXAMPLE36

Paste 1 was prepared by combining 3.0 g of barium sulfate (BaSO₄), 0.18 g of chitosan lactate, and 1.8 g of an aqueous solution (8.5M) of glycolic acid. Paste 2 was composed of 3 g of TTCP and 1.3 mL of water. Approximately equal volumes of the two pastes were dispensed and homogeneously mixed using a dual-barrel micro dispenser (1:1 volume ratio) equipped with a static mixer in the delivery tip. The combined pastes hardened almost immediately.

### EXAMPLE37

Paste 1 was prepared by blending 3g of MCPM into 4g of an aqueous solution containing 8.5 M glycolic acid and 10 wt% chitosan lactate. Paste 2 was composed of 3g Portland cement and 1.2 g of glycerin. Approximately equal volumes of the two pastes were dispensed and homogeneously mixed using a dual-barrel micro dispenser (1:1 volume ratio) equipped with a static mixer in the delivery tip. The combined pastes hardened in 5 minutes at 37 °C. Diametral tensile strength (DTS) of 1-day set cement sample was 2.05 ± 0.32 MPa (n = 5).

### EXAMPLE38

Paste 1 was prepared by blending 3g of MCPM into 4g of an aqueous solution containing 8.5 M glycolic acid and 10 wt% chitosan lactate. Paste 2 was composed of 3g TTCP, 0.015g HPMC, and 1.5 g of glycerin. Approximately equal volumes of the two pastes were dispensed and homogeneously mixed using a dual-barrel micro dispenser (1:1 volume ratio) equipped with a static mixer in the delivery tip. The combined pastes hardened less than 5 minutes at 37 °C.

### EXAMPLE39

Paste 1 was prepared by blending 3g of MCPM into 4g of an aqueous solution containing 8.5M glycolic acid and 10wt% chitosan lactate.. Paste 2 was composed of 3g of a calcium phosphate cement (CPC) mixture (containing 73 wt% TTCP and 27 wt% DCPA) and 1.2 g of glycerin. Approximately equal volumes of the two pastes were dispensed and homogeneously mixed using a dual-barrel micro dispenser (1:1 volume ratio) equipped with a static mixer in the delivery tip. The combined pastes hardened less than 5 minutes at 37 °C.

Examples 40-42 and 43-45 describe calcium phosphate cement systems.

### EXAMPLE 40

Paste 1 was composed of 3.0 g of MCPM, 0.1 g of hydroxypropyl methyl cellulose (HPMC) and 1.5 mL of a solution saturated with respect to both MCPM and DCPD ([Ca] = 1.3 M, [P] = 4.4 M, pH= 1.9). Paste 2 was composed of 3g of a TTCP and 2 g of glycerin. Approximately equal volumes of the two pastes were dispensed and homogeneously mixed using a dual-barrel micro dispenser (1:1 volume ratio) equipped with a static mixer in the delivery tip. The combined pastes set almost instantly at room temperature (21°C).

### EXAMPLE 41

Paste 1 was composed of 3 g of DCPA, 0.1 g of hydroxypropyl methyl cellulose (HPMC) and 2 mL of a 1 M NaH₂PO₄ solution. Paste 2 was composed of 3g of a TTCP and 2 g of glycerin. Approximately equal volumes of the two pastes were dispensed and homogeneously mixed using a dual-barrel micro dispenser (1:1 volume ratio) equipped with a static mixer in the delivery tip. The combined pastes set in approximately 5 minutes at room temperature (21°C).

### EXAMPLE 42

Paste 1 was composed of 3.0 g of MCPM, 0.017 g of hydroxypropyl methyl cellulose (HPMC), 1.5 g of a solution saturated with respect to both MCPM and DCPD ([Ca] = 1.3 M, [P] = 4.4 M, pH= 1.9), and 0.5g of glycerin. Paste 2 was composed of 3g of a calcium phosphate cement (CPC) mixture (containing 73 wt% TTCP and 27 wt% DCPA), 0.017 g of HPMC, and 1.2 g of glycerin. Approximately equal volumes of the two pastes were dispensed and homogeneously mixed using a dual-barrel micro dispenser (1:1 volume ratio) equipped with a static mixer in the delivery tip. The combined pastes hardened less than 10 minutes at 37 °C. Diametral tensile strength of 1-day set cement sample was 2.39 ± 0.60 MPa (n = 5).

### COMPARATIVE EXAMPLE 1

A dual-paste cement identical to the one given in Example 2 of US Patent Application 20040244651 (Lemaitre et al, 2004) was prepared. Paste 1 was composed of 1.36 g of DCPA, 2.3 g of HA (Ca₅(PO₄)₃OH), and 1.85 g of 10 mmol/1 of orthophosphoric acid. Paste 2 of the cement was composed of 3.66 g of TTCP and 1.85 g of sterile water. Approximately equal volumes of the two pastes were dispensed and homogeneously mixed using a dual-barrel micro dispenser (1:1 volume ratio) equipped with a static mixer in the delivery tip. The combined pastes did not set within 60 minutes at 37° C. Because this cement hardened too slowly, another dual paste cement that additionally contained a setting accelerator was described next.

### COMPARATIVE EXAMPLE 2

A dual-paste cement similar to the one given in Example 2 of US Patent Application 20040244651 (Lemaitre et al, 2004) was prepared. Paste 1 was composed of 1.36 g of DCPA, 2.3 g of HA (Ca₅(PO₄)₃OH), and 1.15 g of an aqueous solution (1.5 M) of NaH₂PO₄, a setting accelerator. Paste 2 of the cement was composed of 3.66 g of TTCP and 1.85 g of sterile water. Approximately equal volumes of the two pastes were dispensed and homogeneously mixed using a dual-barrel micro dispenser (1:1 volume ratio) equipped with a static mixer in the delivery tip. The combined pastes set in approximately 25 minutes at 37° C.

The two cement pastes, kept in separate air tight containers, were aged for 7 days at 50 °C. The setting time of cement samples prepared from the aged pastes was greater than 4 days. This result indicates that one or both of the cement pastes had lost reactivity as a result of aging.

### EXAMPLE 43

A dual-paste cement similar to that prepared in Comparative Example 2 was prepared, except that paste 2 was water-free. Paste 1 was composed of 1.36 g of DCPA, 2.3 g of HA (Ca₅(PO₄)₃OH), and 1.15 g of an aqueous solution (1.5 M) of NaH₂PO₄. Paste 2 of the cement was composed of 3.66 g of TTCP and 2.28 g of glycerin. Approximately equal volumes of the two pastes were dispensed and homogeneously mixed using a dual-barrel micro dispenser (1:1 volume ratio) equipped with a static mixer in the delivery tip. The combined pastes set in approximately 25 minutes at 37° C.

The two cement pastes, kept in separate air tight containers, were aged for 7 days at 50° C. The setting time of cement samples prepared from the aged pastes was about 22 minutes. This result indicates that neither cement paste had lost reactivity as a result of aging. By removing water from the second paste, it was seen that the stability of the system was enhanced significantly.

### EXAMPLE 44

Paste 1 was composed of 3.0 g of MCPM, 0.017 g of hydroxypropyl methyl cellulose (HPMC), 1.5 g of a solution saturated with respect to both MCPM and DCPD ([Ca] = 1.3 M, [P] = 4.4 M, pH= 1.9). Paste 2 was composed of 3g of a α-tricalcium phosphate, 0.017 g of HPMC, 0.018 g of HPMC, and 1.74 g of glycerin. Approximately equal volume of the two pastes were dispensed and homogeneously mixed using a dual-barrel micro dispenser (1:1 volume ratio) equipped with a static mixer in the delivery tip. The combined pastes hardened less than 7 minutes at 37 °C. Diametral tensile strength of 1-day set cement sample was 1.31 ± 0.26 MPa (n = 3).

### EXAMPLE45

Paste 1 was composed of 3.0 g of MCPM, 0.017 g of hydroxypropyl methyl cellulose (HPMC), 1.5 g of a solution saturated with respect to both MCPM and DCPD ([Ca] = 1.3 M, [P] = 4.4 M, pH= 1.9). Paste 2 was composed of 3g of a β - tricalcium phosphate, 0.017 g of HPMC, 0.018 g of HPMC, and 1.74 g of glycerin. Approximately equal volume of the two pastes were dispensed and homogeneously mixed using a dual-barrel micro dispenser (1:1 volume ratio) equipped with a static mixer in the delivery tip. The combined pastes hardened less than 7 minutes at 37 °C. Diametral tensile strength of 1-day set cement sample was 2.28 ± 0.27 MPa (n = 5).

It is thus seen that the present invention provides, in various preferred embodiments, dual-phase cement precursor systems, and related kits and methods. The cements of the invention are bio-compatible, in that they are compatible with soft and hard tissues. In preferred embodiments, the cements are osteoconductive and do not cause chronic inflammation of tissues. The cements preferably are injectable, and, when injected, blending is caused during injection, thus permitting the surgeon the maximum amount of time between injection of the cement and hardening of the cement. Through modification of the various cement chemistries employed in connection with the present invention, a range of hardening times, a range of physical and strength properties, and a range of *in vivo* absorption rates may be realized.

## Claims

1. A dual-phase cement precursor system comprising first and second discrete pastes, said first paste comprising a first calcium phosphate compound and said second paste comprising a second calcium phosphate compound, said first and second pastes being separate, said first and second pastes forming a biocompatible cement upon mixing, **characterised in that** one of said first and second pastes being aqueous and the other of said first and second pastes being at least substantially non-aqueous, whereby trace amounts of moisture may be present in said substantially non-aqueous paste.

2. A dual-phase cement precursor system according to claim 1, said first paste comprising a calcium phosphate compound having a Ca/P ratio of less than 5/3 and said second paste comprising a calcium phosphate compound having a Ca/P ratio greater than 5/3.

3. A dual-phase cement precursor system according to claim 2, said first paste including at least one compound selected from the first group consisting of monocalcium phosphate monohydrate, dicalcium phosphate anhydrous, monocalcium phosphate anhydrous, and dicalcium phosphate dihydrate.

4. A dual-phase cement precursor system according to claim 2, said second paste including tetracalcium phosphate.

5. A dual-phase cement precursor system according to any preceding claim, said non-aqueous paste including a liquid selected from glycerin, ethanol, propanol, polyethylene glycol or polypropylene glycol.

6. A dual-phase cement precursor system according to any preceding claim, said non-aqueous paste including glycerin.

7. A dual-phase cement precursor system according to any preceding claim, at least one of said first and second pastes comprising an osteoinductive agent, a radio-opaque filler, macropore-forming agent, a medicament, or a viscosity-enhancing agent.

8. A dual-phase cement precursor system according to any preceding claim, including a strength-enhancing component.

9. A kit comprising the system of any preceding claim and a dispensing device.

10. A dual-phase cement precursor system according to any one of claims 1 to 6 for use in bone repair.

## Patentansprüche

1. Doppelphasen-Zement-Vorläufersystem, umfassend erste und zweite einzelne Pasten, wobei die erste Paste eine erste Calciumphosphat-Verbindung umfasst, und die zweite Paste eine zweite Calciumphosphat-Verbindung umfasst, wobei die erste und zweite Paste getrennt sind, wobei die erste und zweite Paste nach Mischen einen biokompatiblen Zement bilden, **dadurch gekennzeichnet, dass** eine der ersten und zweiten Paste wässrig ist und die andere der ersten und zweiten Paste mindestens im Wesentlichen nicht-wässrig ist, wobei Spurenmengen von Feuchtigkeit in der im Wesentlichen nicht-wässrigen Paste vorliegen können.

2. Doppelphasen-Zement-Vorläufersystem nach Anspruch 1, wobei die erste Paste eine Calciumphosphat-Verbindung mit einem Ca/P-Verhältnis von weniger als 5/3 umfasst und die zweite Paste eine Calciumphosphat-Verbindung mit einem Ca/P-Verhältnis größer als 5/3 umfasst.

3. Doppelphasen-Zement-Vorläufersystem nach Anspruch 2, wobei die erste Paste mindestens eine Verbindung, ausgewählt aus der ersten Gruppe, bestehend aus Monocalciumphosphatmonohydrat, wasserfreiem Dicalciumphosphat, wasserfreiem Monocalciumphosphat und Dicalciumphosphatdihydrat, einschließt.

4. Doppelphasen-Zement-Vorläufersystem nach Anspruch 2, wobei die zweite Paste Tetracalciumphosphat einschließt.

5. Doppelphasen-Zement-Vorläufersystem nach einem vorstehenden Anspruch, wobei die nicht-wässrige Paste eine Flüssigkeit, ausgewählt aus Glycerin, Ethanol, Propanol, Polyethylenglycol oder Polypropylenglycol, einschließt.

6. Doppelphasen-Zement-Vorläufersystem nach einem vorstehenden Anspruch, wobei die nicht-wässrige Paste Glycerin einschließt.

7. Doppelphasen-Zement-Vorläufersystem nach einem vorstehenden Anspruch, wobei mindestens eine der ersten und zweiten Paste ein osteoinduktives Mittel, einen radio-opaken Füllstoff, ein Makroporen-bildendes Mittel, ein Medikament oder ein Viskositäts-verstärkendes Mittel umfasst.

8. Doppelphasen-Zement-Vorläufersystem nach einem vorstehenden Anspruch, das eine Festigkeit-verstärkende Komponente einschließt.

9. Kit, umfassend das System nach einem vorstehenden Anspruch und eine Spender-Vorrichtung.

10. Doppelphasen-Zement-Vorläufersystem nach einem der Ansprüche 1 bis 6 zur Verwendung bei der Knochenreparatur.

## Revendications

1. Système de précurseur de ciment à deux phases comprenant une première et une seconde pâtes distinctes, ladite première pâte comprenant un premier composé de phosphate de calcium et ladite deuxième pâte comprenant un deuxième composé de phosphate de calcium, lesdites première et deuxième pâtes étant séparées, lesdites première et seconde pâtes formant un ciment biocompatible lors du mélange, **caractérisé en ce que** l'une desdites première et seconde pâtes est aqueuse et l'autre desdites première et seconde pâtes est au moins essentiellement non aqueuse, de sorte que des quantités à l'état de traces d'humidité peuvent être présentes dans ladite pâte essentiellement non aqueuse.

2. Système de précurseur de ciment à deux phases selon la revendication 1, ladite première pâte comprenant un composé de phosphate de calcium ayant un rapport Ca/P inférieur à 5/3 et ladite seconde pâte comprenant un composé de phosphate de calcium ayant un rapport Ca/P supérieur à 5/3.

3. Système de précurseur de ciment à deux phases selon la revendication 2, ladite première pâte comprenant au moins un composé choisi parmi le phosphate de monocalcique monohydrate, le phosphate dicalcique anhydre, le phosphate monocalcique anhydre et le phosphate dicalcique dihydrate.

4. Système de précurseur de ciment à deux phases selon la revendication 2, ladite seconde pâte comprenant le phosphate tétracalcique.

5. Système de précurseur de ciment à deux phases selon l'une quelconque des revendications précédentes, ladite pâte non aqueuse comprenant un liquide choisi parmi la glycérine, l'éthanol, le propanol, le polyéthylène glycol ou le polypropylène glycol.

6. Système de précurseur de ciment à deux phases selon l'une quelconque des revendications précédentes, ladite pâte non aqueuse comprenant la glycérine.

7. Système de précurseur de ciment à deux phases selon l'une quelconque des revendications précédentes, l'une au moins desdites première et seconde pâtes comprenant un agent ostéo-inducteur, une charge radio-opaque, un agent de formation de macro-pores, un médicament ou un agent améliorant la viscosité.

8. Système de précurseur de ciment à deux phases selon l'une quelconque des revendications précédentes, comprenant un composant renforçant la résistance.

9. Kit comprenant le système selon l'une quelconque des revendications précédentes et un dispositif de distribution.

10. Système de précurseur de ciment à deux phases selon l'une quelconque des revendications 1 à 6, destiné à être utilisé dans la réparation osseuse.
